(19) Europäisches Patentamt
European Patent Office
Office européen des brevets

(11) **EP 4 717 258 A1**

(12) **EUROPEAN PATENT APPLICATION**
published in accordance with Art. 153(4) EPC

(43) Date of publication:
**01.04.2026  Bulletin 2026/14**

(21) Application number: **24849472.6**

(22) Date of filing: **25.07.2024**

(51) International Patent Classification (IPC):
**A61K 9/16** (2006.01)   **A61K 9/00** (2006.01)
**A61K 38/26** (2006.01)

(52) Cooperative Patent Classification (CPC):
**A61K 9/00; A61K 9/16; A61K 38/26**

(86) International application number:
**PCT/KR2024/010766**

(87) International publication number:
**WO 2025/028903 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.08.2023  KR 20230100675
23.07.2024  KR 20240096968**

(71) Applicant: **Inventage Lab Inc.
Gyeonggi-do 13403 (KR)**

(72) Inventors:
• **KIM, Ju Hee
Seongnam-si, Gyeonggi-do 13494 (KR)**
• **KIM, Min Sung
Seongnam-si, Gyeonggi-do 13494 (KR)**

(74) Representative: **BCKIP Part mbB
MK1
Landsbergerstraße 98, 3.Stock
80339 München (DE)**

(54) **MICROPARTICLES COMPRISING SEMAGLUTIDE OR PHARMACEUTICALLY ACCEPTABLE SALT THEREOF, AND PREPARATION METHOD THEREFOR**

(57)    The present invention relates to microparticles comprising semaglutide or a pharmaceutically acceptable salt thereof, and a preparation method therefor. When the microparticles are injected into the body as an injection, continuous release thereof for 4 weeks or more is possible without initial over-release of semaglutide or a pharmaceutically acceptable salt thereof. In addition, the present invention relates to a method for preparing microparticles having a donut shape in which a hole is formed in the center thereof at the cross-section of the microparticles, and having uniform diameters.

FIG. 2

EP 4 717 258 A1

**Description**

**Technical Field**

The present invention relates to a microparticle containing semaglutide and a method for producing the same.

**Background Art**

Glucagon-like peptide 1 (GLP-1) and analogs and derivatives thereof are highly effective in the treatment of type I and type II diabetes, but their effectiveness is limited due to high clearance rates. The structure of glucagon-like peptide 1 (GLP-1) has been modified using a series of different methods to provide GLP-1 compounds having a longer duration of action *in vivo.*

For example, WO99/43708 discloses GLP-1(7-35) and GLP-1(7-36) derivatives having a lipophilic substituent attached to the C-terminal amino acid residue. WO00/34331 discloses acylated GLP-1 analogs. WO00/69911 discloses activated insulinotropic peptides for injection into patients.

Currently commercially available GLP-1 drugs include, for example, exenatide that is a natural GLP-1 analog which is administered twice daily, liraglutide and lixisenatide, which are administered once daily, and semaglutide, exenatide, albiglutide, dulaglutide, and PEG-loxenatide, which are administered once weekly.

The above-described GLP-1 drugs are mainly used for the treatment of diabetes, and for the treatment of diabetes, the drugs need to be administered in a sustained manner, such as twice daily, once daily, or once weekly, as described above.

In addition, since the above-described GLP-1 drugs have recently been known to have excellent effects not only on the treatment of diabetes but also on the prevention or treatment of obesity, sustained administration of the GLP-1 drugs is required.

Considering this fact, there is a need to develop a formulation that can release semaglutide or a pharmaceutically acceptable salt thereof among GLP-1 drugs in a sustained manner for one month or more by a single administration.

[Prior Art Documents]

[Patent Documents]

(Patent Document 1) KR 10-2022-0170782 A1

**DISCLOSURE**

**Technical Problem**

An object of the present invention is to provide a microparticle containing semaglutide or a pharmaceutically acceptable salt thereof and a method for producing the same.

Another object of the present invention is to provide a microparticle that releases semaglutide or a pharmaceutically acceptable salt thereof in a sustained manner for 4 weeks or more without initial burst release after the microparticle is injected *in vivo.*

Still another object of the present invention is to provide a method for producing microparticles having a donut shape with a hole formed in the center when viewing the cross-section of the microparticle and having a uniform diameter.

**Technical Solution**

To achieve the above-described objects, the present invention relates to a microparticle containing semaglutide or a pharmaceutically acceptable salt thereof, wherein the microparticle comprises semaglutide or the pharmaceutically acceptable salt thereof and a biodegradable polymer, and the cross-section of the microparticle has a shape with a hole formed in the center thereof, and has a value of 0.35 to 0.80 as calculated by Equation 1 below:

$$[\text{Equation 1}]$$

$$(D_{sp} - D_p)/D_{sp}$$

wherein

$D_{sp}$ is the diameter (μm) of the cross-section of the microparticle, and

$D_p$ is the diameter (μm) of the hole formed in the center of the cross-section of the microparticle.

In addition, the microparticle may comprise semaglutide or the pharmaceutically acceptable salt thereof and the biodegradable polymer at a weight ratio of 1:8 to 1:20.

In addition, the biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

The polylactide-co-glycolide may have a monomer ratio of lactide: glycolide of 50:50 to 85:15.

In addition, the biodegradable polymer may have an intrinsic viscosity of 0.1 dl/g to 0.5 dl/g.

In addition, the microparticle may release semaglutide or the pharmaceutically acceptable salt thereof in a sustained manner for 4 weeks or more after being injected *in vivo.*

In addition, the microparticle may have a span value of 1.2 or less.

In addition, the microparticle may have an average diameter of 30 to 70 μm.

To achieve the above-described objects, another aspect of the present invention relates to a method for producing microparticles containing semaglutide or a pharmaceutically acceptable salt thereof, comprising: a step of preparing an oil phase solution containing semaglutide or the pharmaceutically acceptable salt thereof and a biodegradable polymer; a step of preparing a water phase solution by dissolving a surfactant in water; a step of forming an emulsion by mixing the oil phase solution and the water phase solution; a step of collecting the emulsion into the water phase solution to remove residual solvent; and a step of washing and freeze-drying the emulsion from which the residual solvent has been removed, thereby producing the microparticles containing semaglutide or the pharmaceutically acceptable salt thereof, wherein the cross-section of the microparticle has a shape with a hole formed in the center thereof, and has a value of 0.35 to 0.80 as calculated by Equation 1 below:

$$[\text{Equation 1}]$$

$$(D_{sp} - D_p)/D_{sp}$$

wherein

$D_{sp}$ is the diameter (μm) of the cross-section of the microparticle, and

$D_p$ is the diameter (μm) of the hole formed in the center of the cross-section of the microparticle.

In addition, the oil phase solution may be prepared by mixing a first oil phase solution containing semaglutide and a second oil phase solution containing the biodegradable polymer, wherein the first oil phase solution may contain semaglutide or the pharmaceutically acceptable salt thereof and a channeling agent.

In addition, the channeling agent may be selected from the group consisting of methanol, ethanol, propanol, butanol, hydrochloric acid (HCl), nitric acid ($HNO_3$), sulfuric acid ($H_2SO_4$), acetic acid ($CH_3COOH$), boric acid ($H_3BO_3$), carbonic acid ($H_2CO_3$), dimethyl sulfoxide ($C_2H_6OS$), acetonitrile ($C_2H_3N$), ethyl acetate ($C_4H_8O_2$), and mixtures thereof.

In addition, the channeling agent may be contained in an amount of 30 wt% to 60 wt% based on the total weight of the organic solvent of the second oil phase solution.

## Advantageous Effects

When the microparticle containing semaglutide or a pharmaceutically acceptable salt thereof according to the present invention is injected *in vivo,* it may release semaglutide or the pharmaceutically acceptable salt thereof in a sustained manner for 4 weeks or more without initial burst release.

In addition, it is possible to produce microparticles having a donut shape with a hole formed in the center when viewing the cross-section of the microparticle and having a uniform diameter.

## Brief Description of Drawings

FIG. 1 shows SEM images of the cross-sections of microparticles according to one embodiment of the present invention.

FIG. 2 shows SEM images of the cross-sections of microparticles according to one embodiment of the present invention.

FIG. 3 shows SEM images of the cross-sections of microparticles according to one embodiment of the present invention.

FIG. 4 shows SEM images of the cross-sections of microparticles according to one embodiment of the present invention.

FIG. 5 shows SEM images of the cross-sections of microparticles according to one embodiment of the present invention.

FIG. 6 shows the results of measuring the concentration of semaglutide in the blood after administering microparticles according to one embodiment of the present invention to SD rats.

FIG. 7 shows the results of an accelerated release test for microparticles according to one embodiment of the present invention.

FIG. 8 shows an SEM image taken after filling a glass vial with microparticles according to one embodiment of the present invention.

FIG. 9 shows SEM images of the cross-sections of microparticles according to one embodiment of the present invention.

FIG. 10 shows SEM images of the cross-sections of microparticles according to one embodiment of the present invention.

FIG. 11 shows SEM images of the cross-sections of microparticles according to one embodiment of the present invention.

FIG. 12 shows SEM images of the cross-sections of microparticles according to one embodiment of the present invention.

FIG. 13 shows SEM images of the cross-sections of microparticles according to one embodiment of the present invention.

FIG. 14 shows an SEM image of the cross-section of a microparticle according to one embodiment of the present invention.

FIG. 15 shows an SEM image of the cross-section of a microparticle according to one embodiment of the present invention.

FIG. 16 shows the results of measuring the concentration of semaglutide in the blood after administering microparticles according to one embodiment of the present invention to SD rats.

FIG. 17 shows the results of an accelerated release test for microparticles according to one embodiment of the present invention.

FIG. 18 is an SEM image taken after filling a glass vial with microparticles according to one embodiment of the present invention.

**Best Mode**

The present invention relates to a microparticle containing semaglutide or a pharmaceutically acceptable salt thereof, wherein the microparticle comprises semaglutide or the pharmaceutically acceptable salt thereof and a biodegradable polymer, and the cross-section of the microparticle has a shape with a hole formed in the center thereof, and has a value of 0.35 to 0.80 as calculated by Equation 1 below:

$$[\text{Equation 1}]$$

$$(D_{sp} - D_p)/D_{sp}$$

wherein

$D_{sp}$ is the diameter ($\mu$m) of the cross-section of the microparticle, and
$D_p$ is the diameter ($\mu$m) of the hole formed in the center of the cross-section of the microparticle.

## Mode for Invention

Hereinafter, embodiments of the present invention will be described in detail so that those skilled in the art can easily implement the present invention. However, the present invention may be embodied in various different forms and is not limited to the embodiments described herein.

Semaglutide is a drug developed to treat diabetes. It is mainly used to treat type 2 diabetes and is classified as a glucagon-like peptide-1 (GLP-1) receptor agonist. Semaglutide acts similarly to insulin to regulate blood sugar levels and increase insulin secretion from the pancreas, thereby allowing cells to absorb glucose.

This drug is usually administered as a single injection before a meal and is primarily prescribed for the treatment or prevention of diabetes.

Recently, semaglutide has been increasingly used for the prevention and treatment of obesity, taking advantage of its blood sugar-regulating effects. Obesity is closely associated with cardiovascular diseases such as hypertension and diabetes, and obesity management is an important factor in health.

Obesity is one of the most common nutritional disorders worldwide. According to WHO statistics, approximately 250 million people are currently classified as obese, and it is predicted that approximately 300 million people will suffer from obesity in 20 years.

The obesity refers to a state in which body fat is excessively accumulated due to an imbalance in calorie metabolism caused by excessive energy intake or decreased energy consumption. Recently, the incidence of obesity has been rapidly increasing due to the Western-style eating habits and lack of exercise of modern people.

In addition, in terms of numerical concepts, obesity is defined as a body mass index (BMI) of 25 or higher, and the BMI measurement method is an obesity measurement method that estimates the amount of body fat through the value obtained by dividing the weight (kg) by the square ($m^2$) of the height.

Semaglutide, a GLP-1 receptor agonist, is considered a promising candidate for the treatment of obesity due to its appetite-suppressing effects in addition to its blood sugar-regulating effects. Clinical studies have shown that semaglutide may have the effect of inducing weight loss, leading to reduced food intake and reduced total energy intake after a meal.

As described above, obesity requires sustained management, and if semaglutide is to be used for the prevention or treatment of obesity, it needs to be administered in a sustained manner. However, since semaglutide currently needs to be administered once a week, the need for development of a long-acting formulation is increasing.

Accordingly, the present invention relates to a microparticle containing semaglutide or a pharmaceutically acceptable salt thereof, wherein the microparticle comprises semaglutide or the pharmaceutically acceptable salt thereof and a biodegradable polymer, and the cross-section of the microparticle has a shape with a hole formed in the center thereof, and has a value of 0.35 to 0.80 as calculated by Equation 1 below:

$$[\text{Equation 1}]$$

$$(D_{sp} - D_p)/D_{sp}$$

wherein

> $D_{sp}$ is the diameter ($\mu$m) of the cross-section of the microparticle, and
> $D_p$ is the diameter ($\mu$m) of the hole formed in the center of the cross-section of the microparticle.

As described above, the microparticle of the present invention contains semaglutide or a pharmaceutically acceptable salt thereof, and may release semaglutide or the pharmaceutically acceptable salt thereof *in vivo* in a sustained manner for 4 weeks or more by a single administration.

In addition, the microparticle of the present invention is characterized in that the cross-section thereof has a shape with a hole formed in the center as described above, and the cross-sectional portion other than the hole has a plurality of pores formed therein, and the microparticle has a cross-sectional structure as described above.

Equation 1 above relates to the cross-sectional shape of the microparticle of the present invention. To observe the cross-section of the microparticle, the microparticle was cross-sectioned, and for the cross-section of the microparticle, the relationship between $D_{sp}$, which is the diameter of the entire cross-section, and $D_p$, which is the diameter of the hole formed in the center of the cross-section, was expressed. The cross-section has a value of 0.35 to 0.80 as calculated by Equation 1. When the value calculated by Equation 1 is within the range of 0.35 to 0.80 or 0.40 to 0.80, the microparticle may exhibit the effect of releasing semaglutide or the pharmaceutically acceptable salt thereof in a sustained manner for 4 weeks or more without initial burst release, and may exhibit excellent bioavailability (AUC) values, and the encapsulation efficiency of semaglutide or the pharmaceutically acceptable salt thereof in the microparticle may increase, or it is possible to prevent a phenomenon in which the microparticle breaks down when filled into a vial for injection.

If the value calculated by Equation 1 above exceeds the upper limit of the range specified in the present invention, the diameter of the hole formed in the center of the microparticle is short or the hole is not formed, and in this case, there is a problem in that the degree of release of semaglutide or the pharmaceutically acceptable salt thereof is excessively low or the bioavailability (AUC) is excessively low.

In addition, if the value calculated by Equation 1 above is smaller than the lower limit of the range specified in the present invention, problems may arise in that a large hole is formed in the center of the microparticle, making it difficult to encapsulate semaglutide or the pharmaceutically acceptable salt thereof in the microparticle, and when the microparticle is injected *in vivo,* it does not release semaglutide or the pharmaceutically acceptable salt thereof in a sustained manner for 4 weeks or more due to initial burst release, and the microparticle easily breaks down.

The microparticle may contain semaglutide or the pharmaceutically acceptable salt thereof and the biodegradable polymer at a weight ratio of 1:8 to 1:20, specifically 1:9 to 1:19. Only when the microparticle contains semaglutide or the pharmaceutically acceptable salt thereof and the biodegradable polymer at a weight ratio within the above-described range and satisfies the range of the value calculated by Equation 1 above, the microparticle may exhibit the effect of releasing semaglutide or the pharmaceutically acceptable salt thereof in a sustained manner for 4 weeks or more without initial burst release, and exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a vial for injection.

The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and specifically may be polylactide-co-glycolide (PLGA), but the biodegradable polymer is not limited to the above examples and any biodegradable polymer that may contain semaglutide or the pharmaceutically acceptable salt thereof and may release semaglutide or the pharmaceutically acceptable salt thereof *in vivo* in a sustained manner for 4 weeks or more may be used without limitation.

In addition, the polylactide-co-glycolide may have a monomer ratio of lactide: glycolide of 50:50 to 85:15, or 50:50 to 75:25, and an intrinsic viscosity of 0.1 dl/g to 0.5 dl/g, or 0.1 dl/g to 0.4 dl/g, or 0.1 dl/g to 0.3 dl/g. When the microparticle is produced using a selected biodegradable polymer satisfying the above-described conditions, and contains semaglutide or the pharmaceutically acceptable salt thereof and the biodegradable polymer at a weight ratio within the above-described range, and the value calculated by Equation 1 above is within the above-described range, the microparticle may exhibit the effect of releasing semaglutide or the pharmaceutically acceptable salt thereof in a sustained manner for 4 weeks or more without initial burst release, and may exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a vial for injection.

When the biodegradable polymer comprises polylactide-co-glycolide, two or more types of polylactide-co-glycolide having the same viscosity may be used. In this case, the polylactide-co-glycolides may all have the same viscosity, but may have different monomer ratios of lactide: glycolide. For example, polylactide-co-glycolide having a monomer ratio of lactide: glycolide of 50:50 and polylactide-co-glycolide having a monomer ratio of lactide: glycolide of 75:25 may be included at a weight ratio of 1:1 to 1:5, 1:2 to 1:4, or 1:3.

In addition, polylactide-co-glycolide having a monomer ratio of lactide: glycolide of 50:50, polylactide-co-glycolide (PDLG7502A) having a monomer ratio of lactide: glycolide of 75:25, and polylactide-co-glycolide (PDLG7502) having a monomer ratio of lactide: glycolide of 75:25 may be included at a weight ratio of 1:1:2 to 1:1:7, 1:1:3 to 1:1:6, or 1:1:4 to 1:1:6. When the biodegradable polymers are included at a weight ratio within the above range and the value calculated by Equation 1 above is within the above range, the microparticle may exhibit the effect of releasing semaglutide or the pharmaceutically acceptable salt thereof in a sustained manner for 4 weeks or more without initial burst release, and may exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a vial for injection.

The microparticle of the present invention may have a span value of 1.2 or less, 0.7 or less, 0.5 or less, or 0.4 or less. More specifically, the microparticle of the present invention may have a span value of 0.2 to 0.4. Microparticles exhibiting a span value within the above range mean that the diameter of the particles is very uniform. When uniform particles as described above are injected *in vivo,* they may prevent initial burst release of semaglutide or the pharmaceutically acceptable salt thereof and exhibit the effect of releasing semaglutide or the pharmaceutically acceptable salt in a sustained manner for 4 weeks or more.

The microparticle may have an average diameter of 30 to 70 $\mu$m, 30 to 60 $\mu$m, or 30 to 50 $\mu$m. In addition, the standard deviation for the average diameter may be 1 to 30 $\mu$m, 1 to 20 $\mu$m, 1 to 10 $\mu$m, or 1 to 7 $\mu$m. It can be confirmed that uniform particles may be produced within the above diameter range. When the uniform particles are used as a sustained-release injection, foreign body sensation may be reduced, which may improve the convenience of administration, initial burst release after *in vivo* injection may be prevented, and the particles may exhibit the effect of releasing semaglutide or the pharmaceutically acceptable salt thereof in a sustained manner.

A method for producing microparticles containing semaglutide or a pharmaceutically acceptable salt thereof according to another embodiment of the present invention may comprise: a step of preparing an oil phase solution containing semaglutide or the pharmaceutically acceptable salt thereof and a biodegradable polymer; a step of preparing a water phase solution by dissolving a surfactant in water; a step of forming an emulsion by mixing the oil phase solution and the water phase solution; a step of collecting the emulsion into the water phase solution to remove residual solvent; and a step of washing and freeze-drying the emulsion from which the residual solvent has been removed, thereby producing the microparticles containing semaglutide or the pharmaceutically acceptable salt thereof, wherein the cross-section of the microparticle has a shape with a hole formed in the center thereof, and has a value of 0.35 to 0.80 as calculated by Equation 1 below:

[Equation 1]

$$(D_{sp} - D_p)/D_{sp}$$

wherein

$D_{sp}$ is the diameter ($\mu$m) of the cross-section of the microparticle, and
$D_p$ is the diameter ($\mu$m) of the hole formed in the center of the cross-section of the microparticle.

The oil phase solution may be prepared by mixing a first oil phase solution containing semaglutide and a second oil phase solution containing the biodegradable polymer, wherein the first oil phase solution may contain semaglutide or the pharmaceutically acceptable salt thereof and a channeling agent.

The biodegradable polymer included in the second oil phase solution is as described above, and the weight ratio between semaglutide or the pharmaceutically acceptable salt thereof and the biodegradable polymer contained in the oil phase solution prepared by mixing the first oil phase solution and the second oil phase solution is also as described above.

The oil phase solution may be prepared by mixing a first oil phase solution containing semaglutide and a second oil phase solution containing the biodegradable polymer, wherein the first oil phase solution may contain semaglutide or the pharmaceutically acceptable salt thereof and a channeling agent.

Specifically, the channeling agent may be selected from the group consisting of methanol, ethanol, propanol, butanol, hydrochloric acid (HCl), nitric acid ($HNO_3$), sulfuric acid ($H_2SO_4$), acetic acid ($CH_3COOH$), boric acid ($H_3BO_3$), carbonic acid ($H_2CO_3$), dimethyl sulfoxide ($C_2H_6OS$), acetonitrile ($C_2H_3N$), ethyl acetate ($C_4H_8O_2$), and mixtures thereof, and more specifically may be a mixture of methanol and acetic acid. However, the channeling agent is not limited to the above examples, and any channeling agent that may form a hole in the center of the cross-section of the microparticle may be used without limitation.

When methanol and acetic acid are used in combination as the channeling agent as described above, they may be contained at a weight ratio of 1:5 to 1:23, 1:6 to 1:20, or 1:6 to 1:18. When methanol and acetic acid are used in combination at a weight ratio within the above range, the content of the channeling agent based on the total weight of the organic solvent of the second oil phase solution is within the range described below, the weight ratio between the biodegradable polymer and semaglutide or the pharmaceutically acceptable salt thereof is within the above-described range, the type and intrinsic viscosity of the biodegradable polymer are within the above-described ranges, and the value calculated by Equation 1 is within the above-described range, the microparticle may exhibit the effect of releasing semaglutide or the pharmaceutically acceptable salt thereof in a sustained manner for 4 weeks or more without initial burst release, and may exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a vial for injection.

The channeling agent may be contained in an amount of 30 to 60 wt% based on the total weight of the organic solvent of the second oil phase solution. More specifically, the solvent of the second oil phase solution may be dichloromethane (DCM). In the above-described production method, the oil phase solution is prepared by mixing the first oil phase solution and the second oil phase solution. When the first oil phase solution and the second oil phase solution are mixed, the ratio of the weight of the channeling agent contained in the first oil phase solution to the weight of dichloromethane contained in the second oil phase solution may be 30 wt% to 60 wt%. When the weight ratio between dichloromethane and the channeling agent for preparing the above-described oil phase solution satisfies the above-described range, the channeling agent is also contained in an amount within the above-described range, the weight ratio between the biodegradable polymer and semaglutide or the pharmaceutically acceptable salt thereof is within the above-described range, the type and intrinsic viscosity of the biodegradable polymer are within the above-described ranges, and the value calculated by Equation 1 is within the above-described range, the microparticle may exhibit the effect of releasing semaglutide or the pharmaceutically acceptable salt thereof in a sustained manner for 4 weeks or more without initial burst release, and may exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a glass vial for injection.

That is, when the microparticle produced while satisfying all of the conditions described above and the value calculated by Equation 1 above is in the range of 0.35 to 0.80 or 0.40 to 0.80, the microparticle may exhibit the effect of releasing semaglutide or the pharmaceutically acceptable salt thereof in a sustained manner for 4 weeks or more without initial burst release, and may exhibit an excellent bioavailability (AUC) value, and the encapsulation efficiency of semaglutide or the pharmaceutically acceptable salt thereof in the microparticle may increase, or it is possible to prevent a phenomenon in which the microparticle breaks down when filled into a glass vial for injection.

The surfactant in the water phase solution may be contained in an amount of 0.1 to 1.0 wt%, 0.2 to 0.5 wt%, or 0.25 wt%. The remainder is water.

The surfactant may be any one or more selected from the group consisting of methylcellulose, polyvinylpyrrolidone, lecithin, gelatin, polyvinyl alcohol, sorbitan monooleate (e.g., Span™ 80, etc.), polyoxyethylene sorbitan fatty acid ester (e.g., Tween™ 80, etc.), polyoxyethylene castor oil derivatives, sodium lauryl sulfate, sodium stearate, ester amines, linear diamines, patty amines, and mixtures thereof, and preferably may be polyvinyl alcohol, but the surfactant is not limited to the above examples, and any surfactant that may be prepared into a perfectly spherical emulsion may be used.

In addition, the water phase solution may further contain sodium chloride in addition to the surfactant.

In addition, the water phase may be adjusted to a pH of 2 to 4, 2 to 3.5, or 2.5 to 3.0 using an acidic solution.

After the oil phase solution and the water phase solution are separately prepared as described above, the method of preparing an emulsion using the same is not limited.

In the present invention, a method of producing microparticles using a microfluidic method will be described.

A microchip for using the microfluidic method may be formed on a wafer or a glass substrate. Microchannels are formed in the microchip. More specifically, the microchannels include a channel through which the oil phase solution flows, a channel through which the water phase solution flows, and a transport channel. The channel through which the oil phase solution flows and the channel through which the water phase solution flows are formed to meet each other at one point, and one end of the transport channel may be connected to a portion where the two channels are joined.

Injection portions for injecting the oil phase solution and the water phase solution are connected to the channel through which the oil phase solution flows and the channel through which the water phase solution flows, respectively, and a recovery portion for recovering a solution containing an emulsion may be connected to one end of the transport channel.

The microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

As the microchannel for producing the microparticles of the present invention, a 144-channel chip may be used. In the case of the microchip, the average diameter of the microchannels into which the oil phase solution and the water phase solution are to be injected is 300 $\mu$m to 500 $\mu$m, and the oil phase solution and the water phase solution may pass through resistance channels after moving through the respective channels. The average diameter of the resistance channels is 10 $\mu$m to 50 $\mu$m. After passing through the resistance channels, the oil phase solution and the water phase solution pass through a junction channel where they intersect, and the diameter of the junction channel may be 100 $\mu$m to 400 $\mu$m. After the oil phase solution and the water solution intersect each other within the junction channel to form an emulsion, they pass through a microchannel with a diameter of 150 $\mu$m to 200 $\mu$m, and then pass through a microchannel with a diameter of 200 $\mu$m to 300 $\mu$m.

However, the average diameter of the microchannels may vary depending on the range of injection pressure and flow rate conditions. In addition, the average diameter of the microchannels is closely related not only to the average diameter of the particles, but is also to the injection pressure and flow rate conditions of the oil phase solution and the water phase solution.

The oil phase solution and water phase solution prepared as described above may be allowed to flow under the flow rate conditions described below through a first microchannel and a second microchannel, which have an intersection formed therebetween.

That is, the oil phase solution flows along the first microchannel, and the water phase solution flows along the second microchannel configured to form an intersection with the first microchannel, and meets the flow of the oil phase solution.

More specifically, the oil phase solution may be injected into the first microchannel at a flow rate of 200 μL/min to 400 μL/min, or 250 μL/min to 300 μL/min.

In addition, the water phase solution may be injected into the second microchannel at a flow rate of 8,000 μL/min to 30,000 μL/min, or 10,000 μL/min to 15,000 μL/min.

As the flow rates of the oil phase solution and the water phase solution are made different from each other as described above and the flow rate of the water phase solution is increased compared to the flow rate of the oil phase solution, the water phase solution having a relatively higher flow rate compresses the oil phase solution at the point where the flow of the oil phase solution and the flow of the water phase solution meet each other, and in this case, due to repulsive force between the oil phase solution and the water phase solution, the biodegradable polymer and semaglutide or the pharmaceutically acceptable salt thereof in the oil phase solution form spherical emulsion particles containing them, and more specifically, form an emulsion in which semaglutide or the pharmaceutically acceptable salt thereof is uniformly distributed in the spherical biodegradable polymer.

The temperature at which the oil phase solution and the water phase solution flow through the microchannels is also 15 to 20°C, preferably 17°C. That is, after the oil phase solution and the water phase solution flow through the microchannels and intersect each other to produce microparticles, they are constantly maintained at a low temperature of 15 to 20°C until the collected emulsion is stirred. It is possible to produce and maintain spherical particles only when the process of producing microparticles is maintained at low temperature. That is, if a low-temperature condition is not used, a problem arises in that it is difficult to produce particles having a uniform spherical shape.

The produced emulsion may be collected in a bath containing the water phase solution to prevent aggregation of initially produced emulsion particles.

The water phase solution is a water phase solution prepared for the preparation of the emulsion, that is, a mixed solution of the surfactant and purified water. Specifically, a portion of the prepared water phase solution is injected into the microchannel, and the other portion may be transferred into the bath and used to prevent aggregation of the collected emulsion particles.

Thereafter, the organic solvent present in the emulsion collected in the bath may be evaporated and removed by stirring at a predetermined stirring speed at a predetermined temperature. Here, the stirring may be performed at a speed of 200 rpm 400 rpm at 30°C to 40°C for 2 hours to 4 hours. Specifically, the stirring may be performed at a speed of 250 rpm 350 rpm at 35°C to 40°C for 2.5 hours to 3.5 hours.

As the residual organic solvent is removed under the above-described stirring conditions, formation of pores on the surface of the emulsion particles may be prevented, enabling the production of microparticles with a smooth surface, and the residual organic solvent may also be minimized.

Lastly, the emulsion from which the residual organic solvent has been completely removed by stirring may be washed several times with sterile filtered purified water to remove the surfactant remaining thereon, and then freeze-dried, thereby producing microparticles.

The microparticle finally produced is in a form in which semaglutide or the pharmaceutically acceptable salt thereof is uniformly distributed in the microparticle composed of the spherical biodegradable polymer, and the cross-section of the microparticle has a shape with a hole formed in the center thereof. Also, the microparticle may contain semaglutide or the pharmaceutically acceptable salt thereof and the biodegradable polymer at a weight ratio of 1:8 to 1:20.

The weight ratio between semaglutide or the pharmaceutically acceptable salt thereof and the biodegradable polymer contained in the microparticle is the same as the weight ratio between semaglutide or the pharmaceutically acceptable salt thereof and the biodegradable polymer contained in the first oil phase solution and second oil phase solution prepared to prepare the oil phase solution. That is, as the microparticles are produced and the organic solvent is completely removed by evaporation therefrom, microparticles containing semaglutide or the pharmaceutically acceptable salt thereof and the biodegradable polymer at the same weight ratio as that in the oil phase solution may be produced.

The produced microparticles may be prepared into an injectable composition by mixing with a suspending solvent. The suspending solvent contains an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and is preferably D-mannitol, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, but the suspending agent is not limited to the above examples.

As the solvent, water for injection may be used, and any solvent that may be used as water for injection may be used without limitation.

Production Example 1

A first oil phase solution was prepared by dissolving semaglutide in acetic acid and methanol, which are channeling agents. A second oil phase solution was prepared by dissolving PDLG5002A, PDLG7502A, and PDLG7502, which have a viscosity of 0.20 dl/g, in dichloromethane, and this was mixed with the first oil phase solution, thereby preparing an oil phase solution. Here, the weight ratio between semaglutide and the biodegradable polymers in the oil phase solution was 1:11, the weight ratio between the three biodegradable polymers was 1.5:1.5:7.0, and the weight of the channeling agents was 26.5% of the total weight of dichloromethane and the channeling agents. Polyvinyl alcohol, a surfactant, was mixed with water to make an aqueous solution containing 0.5 wt% of polyvinyl alcohol, and then 0.2 wt% of sodium chloride was added thereto, and the pH was adjusted to 2.5 with hydrochloric acid, thereby preparing a water phase solution. The oil phase solution and the water phase solution were injected into microchannels formed on a silicon wafer and allowed to flow. Here, the ratio of the flow rate of the oil phase solution to that of the water phase solution was 1:40. The temperature condition was maintained at 17°C, and the stirring speed was 150 rpm. Microparticles produced at the intersection between the flow of the oil phase solution and the flow of the water phase solution were collected in a bath containing the water phase solution. The microparticles collected in the bath were subjected to first stirring at a speed of 150 rpm for 30 min at 17°C, and then subjected to stirring at a speed of 300 rpm for 3 hours at an increased temperature of 36°C. After completion of stirring, the microparticles were washed several times with sterile filtered purified water and freeze-dried, thereby producing microparticles.

Production Example 2

Microparticles were produced in the same manner as in Production Example 1, except that the weight of the channeling agents was 33.7% of the total weight of dichloromethane and the channeling agents.

Production Example 3

Microparticles were produced in the same manner as in Production Example 1, except that the weight of the channeling agents was 42.2% of the total weight of dichloromethane and the channeling agents.

Production Example 4

Microparticles were produced in the same manner as in Production Example 1, except that the weight of the channeling agents was 56.6% of the total weight of dichloromethane and the channeling agents.

Production Example 5

Microparticles were produced in the same manner as in Production Example 1, except that the weight of the channeling agents was 63.9% of the total weight of dichloromethane and the channeling agents.

Production Example 6

A first oil phase solution was prepared by dissolving semaglutide in acetic acid and methanol, which are channeling agents. A second oil phase solution was prepared by dissolving PLGA5002A and PDLG7502A, which have a viscosity of 0.20 dl/g, in dichloromethane, and the second oil phase solution was mixed with the first oil phase solution, thereby preparing an oil phase solution. Here, the weight ratio between semaglutide and the biodegradable polymers in the oil phase solution was 1:5, the weight ratio between PLGA5050 and PLGA7525 was 1:3, and the weight of the channeling agents was 45.1% of the total weight of dichloromethane and the channeling agents. 0.5 wt% of polyvinyl alcohol, a surfactant, was mixed with water, and then 0.2 wt% of sodium chloride was added thereto, and the pH was adjusted to 3.0 with hydrochloric acid, thereby preparing a water phase solution. The oil phase solution and the water phase solution were injected into microchannels formed on a silicon wafer and allowed to flow. Here, the ratio of the flow rate of the oil phase solution to that of the water phase solution was 1:48. The temperature condition was maintained at 17°C. The emulsion produced at the intersection between the flow of the oil phase solution and the flow of the water phase solution was collected in a bath containing the water phase solution. The emulsion collected in the bath was subjected to first stirring at a speed of 150 rpm for 30 min at 17°C, and then subjected to stirring at a speed of 300 rpm for 3 hours at an increased temperature of 36°C, thereby removing residual organic solvent. The emulsion from which the residual organic solvent was removed was washed several times with sterile filtered purified water and freeze-dried, thereby producing microparticles.

Production Example 7

Microparticles were produced in the same manner as in Production Example 6, except that the weight ratio between semaglutide and the biodegradable polymers in the oil phase solution was 1:9, and the weight of the channeling agents was 43.7% of the total weight of dichloromethane and the channeling agents.

Production Example 8

Microparticles were produced in the same manner as in Production Example 6, except that the weight ratio between semaglutide and the biodegradable polymers in the oil phase solution was 1:11, and the weight of the channeling agents was 42.2% of the total weight of dichloromethane and the channeling agents.

Production Example 9

Microparticles were produced in the same manner as in Production Example 6, except that the weight ratio between semaglutide and the biodegradable polymers in the oil phase solution was 1:15, and the weight of the channeling agents was 49.6% of the total weight of dichloromethane and the channeling agents.

Production Example 10

Microparticles were produced in the same manner as in Production Example 6, except that the weight ratio between semaglutide and the biodegradable polymers in the oil phase solution was 1:19, and the weight of the channeling agents was 46.1% of the total weight of dichloromethane and the channeling agents.

Production Example 11

Microparticles were produced in the same manner as in Production Example 6, except that the weight ratio between semaglutide and the biodegradable polymers in the oil phase solution was 1:22, and the weight of the channeling agents was 44.5% of the total weight of dichloromethane and the channeling agents.

Production Example 12

Microparticles were produced in the same manner as in Production Example 6, except that the weight ratio between semaglutide and the biodegradable polymers in the oil phase solution was 1:34, and the weight of the channeling agents was 40.3% of the total weight of dichloromethane and the channeling agents.
Specific conditions for the oil phase solution and water phase solution of each of Production Examples 1 to 12 are as shown in Table 1 below:

[Table 1]

| No. | Oil phase solution | | | | | | | Water phase solution | |
|---|---|---|---|---|---|---|---|---|---|
| | A:P ratio | Polymers | | Channeling agents | | Solvent | Polymers | Inside | Outside |
| | | Type and ratio | Amount used (g) | Acetic acid (g) | Methanol (g) | Dichloromet hane (g) | Concentratio n (%) | | |
| Production Example 1 | 1:11 | PDLG5002A(1.5)+PDL G7502A(1.5)+PDLG750 2(7.0) | 1.10 | 1.70 | 0.50 | 6.10 | 11.7 | 0.5% PVA IL + NaCl 2g by HCl (pH2.5) | |
| Production Example 2 | | | 1.10 | 2.30 | 0.50 | 5.50 | 11.7 | | |
| Production Example 3 | | | 1.10 | 3.00 | 0.50 | 4.80 | 11.7 | | |
| Production Example 4 | | | 1.10 | 4.20 | 0.50 | 3.60 | 11.7 | | |
| Production Example 5 | | | 1.10 | 4.80 | 0.50 | 3.00 | 11.7 | | |
| Production Example 6 | 1:5 | PDLG5002A(1) + PDLG7502A(3) | 0.50 | 1.80 | 0.25 | 2.50 | 9.9 | 0.5% PVA 1L + NaCl 2g by HCl (pH3.0) | |
| Production Example 7 | 1:9 | | 0.45 | 1.30 | 0.25 | 2.00 | 11.3 | | |
| Production Example 8 | 1:11 | | 0.55 | 1.50 | 0.25 | 2.40 | 11.7 | | |
| Production Example 9 | 1:15 | | 0.75 | 3.00 | 0.25 | 3.30 | 10.3 | | |
| Production Example 10 | 1:19 | | 0.95 | 3.25 | 0.25 | 4.10 | 11.1 | | |
| Production Example 11 | 1:22 | | 1.10 | 3.60 | 0.25 | 4.80 | 11.3 | | |
| Production Example 12 | 1:34 | | 1.70 | 4.30 | 0.25 | 6.75 | 13.1 | | |

Here, A:P refers to the weight ratio of semaglutide and biodegradable polymer.

The conditions for producing microparticles by a microfluidic method using the oil phase solution and water phased solution of each of Production Examples 1 to 12 are specifically as shown in Table 2 below:

[Table 2]

| No. | Production conditions | | | | | Solvent removal | | |
|---|---|---|---|---|---|---|---|---|
| | Integrated chip (channel) | Flow rate conditions | | Temperature (°C) | Stirring (rpm) | Temperature (°C) | Stirring (rpm) | Time (h) |
| | | Oil phase ($\mu$L/min) | Water phase ($\mu$L/min) | | | | | |
| Production Example 1 | 144 | 300 | 12,000 | 17 | 150 | 36 | 300 | 3 |
| Production Example 2 | | | | | | | | |
| Production Example 3 | | | | | | | | |
| Production Example 4 | | | | | | | | |
| Production Example 5 | | | | | | | | |
| Production Example 6 | 144 | 250 | 12,000 | 17 | 150 | 36 | 300 | 3 |
| Production Example 7 | | | | | | | | |
| Production Example 8 | | | | | | | | |
| Production Example 9 | | | | | | | | |
| Production Example 10 | | | | | | | | |
| Production Example 11 | | | | | | | | |
| Production Example 12 | | | | | | | | |

Test Example 1

Observation of Cross-Sections of Microparticles and pK Test Results

The microparticles of Production Examples 1 to 5 were cross-sectioned, and SEM images were taken of the cross-sections of the microparticles to measure the overall diameter ($\mu$m) of the cross-section of the microparticle and, if a hole was formed in the center, the diameter ($\mu$m) of the hole.

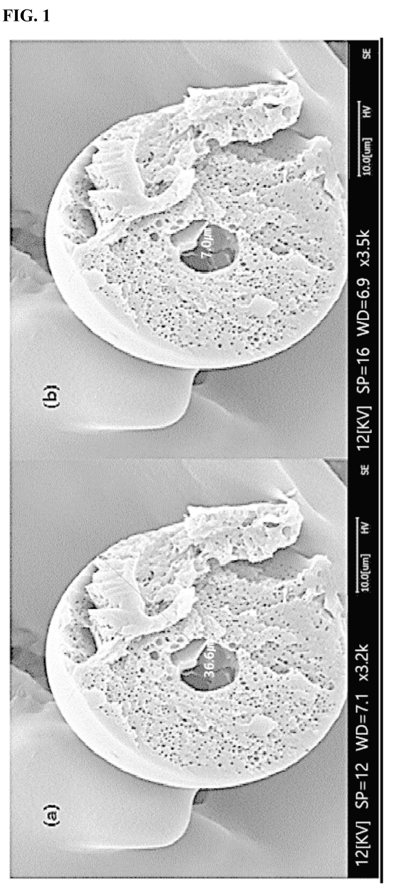

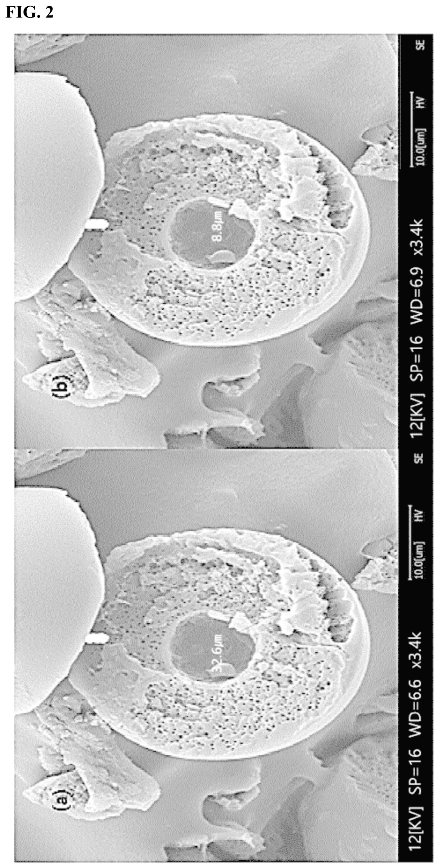

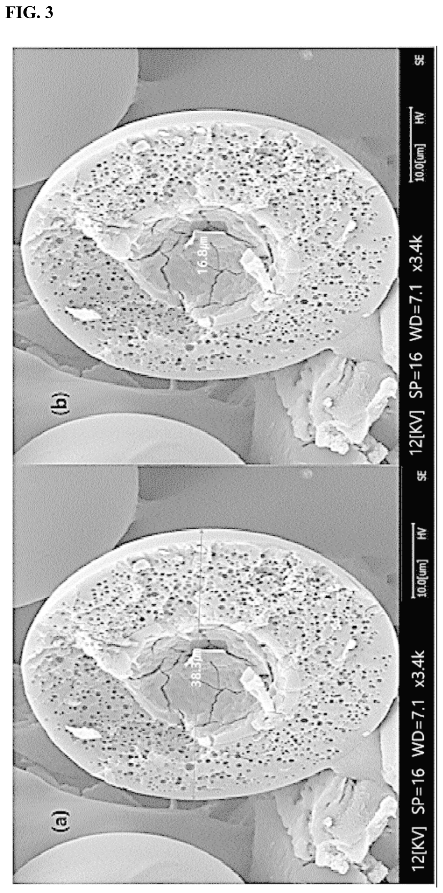

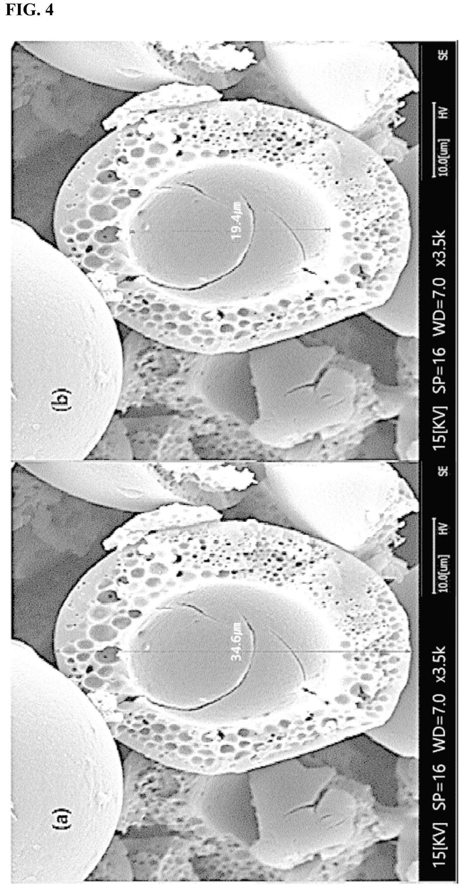

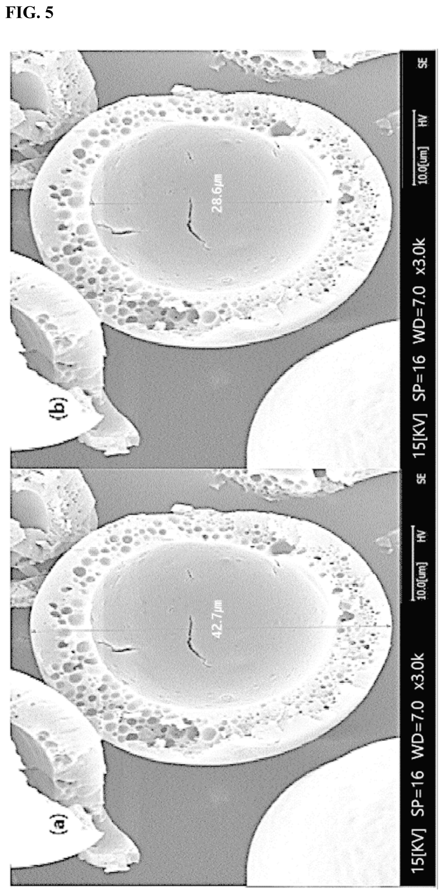

SEM images of the cross-sections for Production Examples 1 to 5 are as shown in FIGS. 1 to 5. When examining the cross-sections in FIGS. 1 to 5, it can be confirmed that a hole was formed in the center.

The results of measuring the diameter for Production Examples 1 to 5 are shown in Table 3 below:

[Table 3]

| | Production Example 1 | Production Example 2 | Production Example 3 | Production Example 4 | Production Example 5 |
|---|---|---|---|---|---|
| Channeling agents (g) | 2.20 | 2.80 | 3.50 | 4.70 | 5.30 |

(continued)

|  | Production Example 1 | Production Example 2 | Production Example 3 | Production Example 4 | Production Example 5 |
|---|---|---|---|---|---|
| DCM (g) | 6.10 | 5.50 | 4.80 | 3.60 | 3.00 |
| **Weight (%) of channeling agents\*** | **26.5** | **33.7** | **42.2** | **56.6** | **63.9** |
| Cross-sectional diameter (μm) | 36.6 | 32.6 | 38.3 | 34.6 | 42.7 |
| Inner diameter (μm) | 7.0 | 8.8 | 16.8 | 19.4 | 28.6 |
| Value of [Equation 1] | 0.809 | 0.730 | 0.561 | 0.439 | 0.330 |

\* is the weight (%) of the channeling agents relative to the total weight of dichloromethane and the channeling agents. The value of Equation 1 is a value calculated by Equation 1 below:

[Equation 1]

$$(D_{sp} - D_p)/D_{sp}$$

wherein

$D_{sp}$ is the diameter (μm) of the cross-section of the microparticle, and
$D_p$ is the diameter (μm) of the hole formed in the center of the cross-section of the microparticle.

For Production Example 3, pharmacokinetic evaluation was conducted to determine the release of semaglutide. The test conditions are as follows:

- Male SD rats, 7 weeks old
- Ten SD rats were used, and the average value of the blood semaglutide concentrations was calculated for each measurement time.
- Subcutaneous administration (2.8 mg/kg)

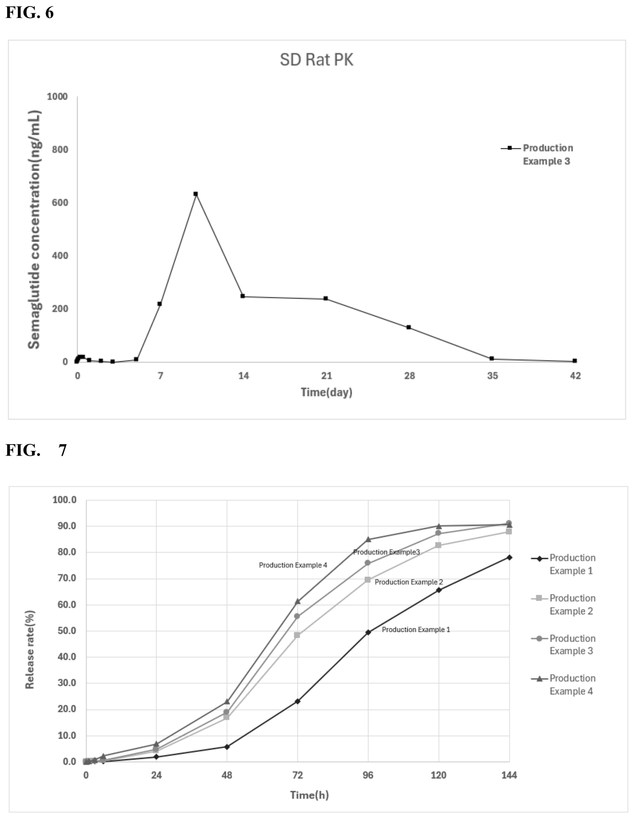

The test results are shown in Table 4 below and FIG. 6:

[Table 4]

| Time (day) | Time (hour) | Production Example 3 |
|---|---|---|
|  |  | (42.2%) |
| 0 | 0 | ND |
| 0.04 | 1 | 3.8494 |
| 0.13 | 3 | 11.5346 |
| 0.25 | 6 | 15.8705 |
| 0.50 | 12 | 16.7602 |
| 1 | 24 | 5.6360 |
| 2 | 48 | 0.9561 |
| 3 | 72 | 0.2567 |
| 5 | 120 | 8.3206 |
| 7 | 168 | 215.8280 |
| 10 | 240 | 630.1975 |
| 14 | 336 | 245.1624 |
| 21 | 504 | 236.5427 |
| 28 | 672 | 127.7270 |
| 35 | 840 | 10.4250 |
| 42 | 1008 | 1.9027 |

(continued)

| Time (day) | Time (hour) | Production Example 3 |
|---|---|---|
| | | (42.2%) |
| AUC$_{last}$ (h·ng/mL) | | 154379.972 |

Referring to the above test results, it was confirmed that, in the case of Production Example 3, initial burst release of semaglutide did not occur, the release of semaglutide gradually increased from the initial stage after administration, and the peak blood concentration of semaglutide appeared around day 10, and then the release of semaglutide gradually decreased, and semaglutide was released until day 42.

Based on the pK test results for Production Example 3 as described above, an accelerated release test was conducted on other Production Examples.

For the accelerated release test, microparticles with a dose equivalent to 5 mg semaglutide were used, and a release test solution was prepared by mixing 1X PBS buffer (pH 7.4), 0.06% (w/v) polysorbate 80, and 0.01% (w/v) sodium azide.

A shaking water bath was used as a dissolution tester, and a glass test vessel with a capacity of 120 ml was used as a dissolution test vessel. As operating conditions, shaking was performed horizontally in a direction perpendicular to the bottom of the vessel and performed 120 times (reciprocating) at 40°C.

The test results are as shown in Table 5 below and FIG. 7:

[Table 5]

| Time (hour) | Production Example 1 | Production Example 2 | Production Example 3 | Production Example 4 |
|---|---|---|---|---|
| 0 | 0.0 | 0.0 | 0.0 | 0.0 |
| 1 | 0.1 | 0.2 | 0.1 | 0.2 |
| 3 | 0.2 | 0.2 | 0.3 | 0.8 |
| 6 | 0.2 | 0.4 | 0.6 | 2.3 |
| 24 | 1.9 | 4.2 | 4.8 | 6.9 |
| 48 | 5.8 | 16.8 | 19.0 | 23.1 |
| 72 | 23.2 | 48.3 | 55.5 | 61.4 |
| 96 | 49.4 | 69.5 | 75.8 | 85.0 |
| 120 | 65.6 | 82.7 | 87.2 | 90.1 |
| 144 | 78.2 | 87.9 | 91.1 | 90.6 |

Production Example 3 showed a low release rate similar to the PK release pattern in the initial stage after administration, and showed a pattern in which the drug began to be released significantly after 24 hours and the release rate of semaglutide continuously increased.

Production Examples 2 and 4 above showed release rates and release patterns similar to Production Example 3, and based thereon, it can be predicted that the microparticles have similar bioavailability and drug release patterns in *in vivo* tests.

On the other hand, the microparticles of Production Example 1 showed a lower release rate than those of Production Examples 2 to 4, and the point in time when the drug was released significantly was relatively late. Based thereon, it can be predicted that the point in time when the drug was released significantly in an *in vivo* test would be late, and bioavailability would also be lower.

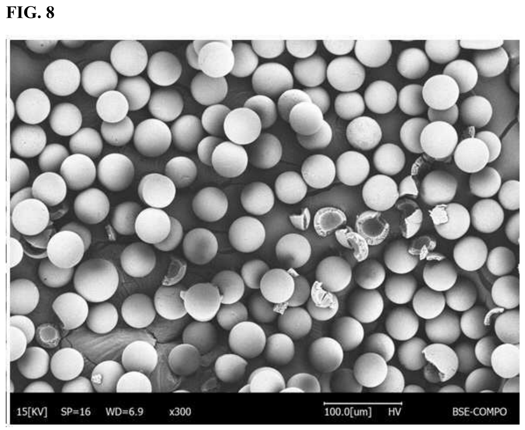

On the other hand, in the case of Production Example 5, despite the fact that semaglutide used in the process of preparing the oil phase solution exceeded 30 wt%, the encapsulation efficiency of semaglutide in the microparticles was low at 73.7%. In addition, because the amount of channeling agents used was large, the value of Equation 1 was 0.330, and thus the diameter of the hole in the microparticle was excessively large compared to the diameter of the cross-section, so that the microparticles easily broke down during the filling process as shown in FIG. 8. The broken microparticles can significantly affect the release pattern of semaglutide when administered *in vivo*.

The results of analyzing the particle sizes of the microparticles of Production Examples 1 to 5 are shown in Table 6 below.

[Table 6]

|  | Production Example 1 | Production Example 2 | Production Example 3 | Production Example 4 | Production Example 5 |
|---|---|---|---|---|---|
| D10 | 32.22 | 31.35 | 31.19 | 31.26 | 36.12 |
| D20 | 33.44 | 32.09 | 32.56 | 32.93 | 37.68 |
| D30 | 34.54 | 32.76 | 33.70 | 34.36 | 38.77 |
| D40 | 35.60 | 33.35 | 34.81 | 35.73 | 39.71 |
| D50 | 36.70 | 33.94 | 35.96 | 37.16 | 40.66 |
| D60 | 37.89 | 34.56 | 37.20 | 38.76 | 41.63 |
| D70 | 39.20 | 35.18 | 38.73 | 40.56 | 42.73 |
| D80 | 40.69 | 35.92 | 40.61 | 42.79 | 43.95 |
| D90 | 42.58 | 36.76 | 43.25 | 46.27 | 46.38 |
| D95 | 43.82 | 37.71 | 45.57 | 49.80 | 48.55 |
| Span value | 0.28 | 0.16 | 0.34 | 0.40 | 0.25 |

Referring to the particle size analysis results in Table 5 above, it can be confirmed that the microparticles produced according to the production method of the present invention are very uniform particles with D50 values of 36.70 μm, 33.94 μm, 35.96 μm, 37.16 μm, and 40.66 μm, and span values of 0.28, 0.16, 0.34, 0.40, and 0.25.

Test Example 2

Observation of Cross-Sections of Microparticles and pK Test Results

The microparticles of Production Examples 6 to 12 were cross-sectioned, and SEM images were taken of the cross-sections of the microparticles to measure the overall diameter (μm) of the cross-section of the microparticle and, if a hole was formed in the center, the diameter (μm) of the hole.

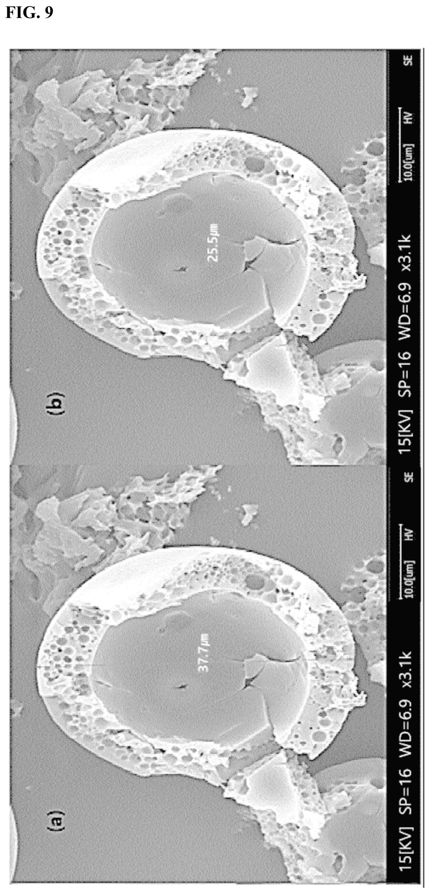

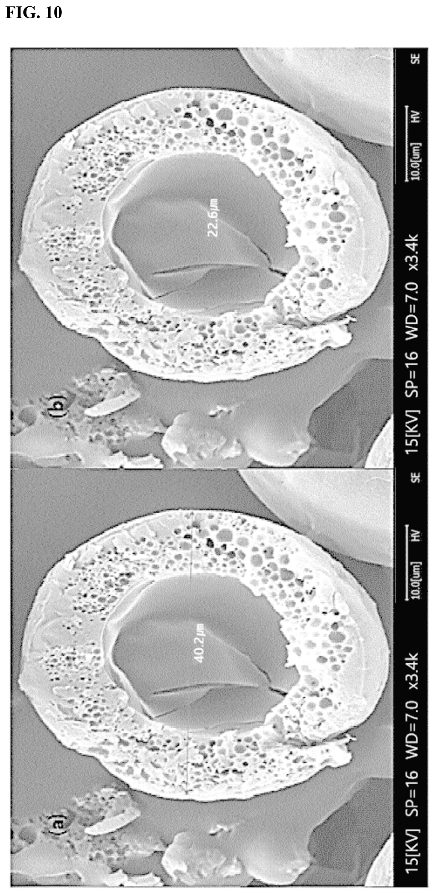

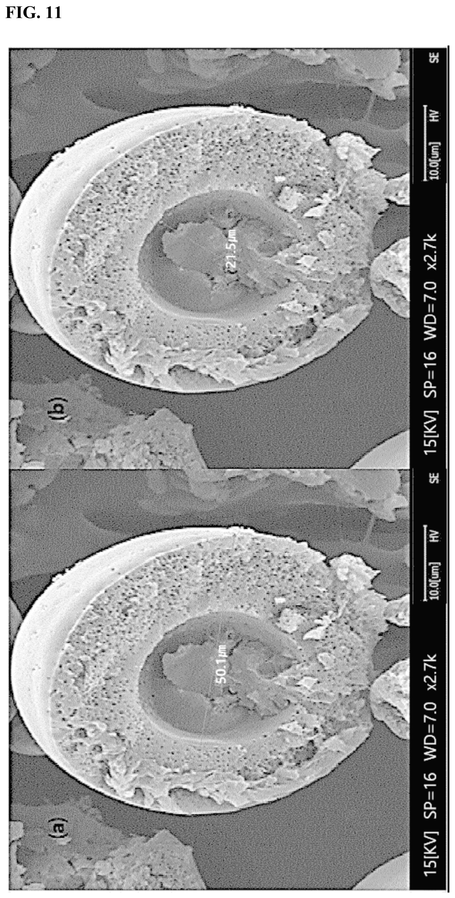

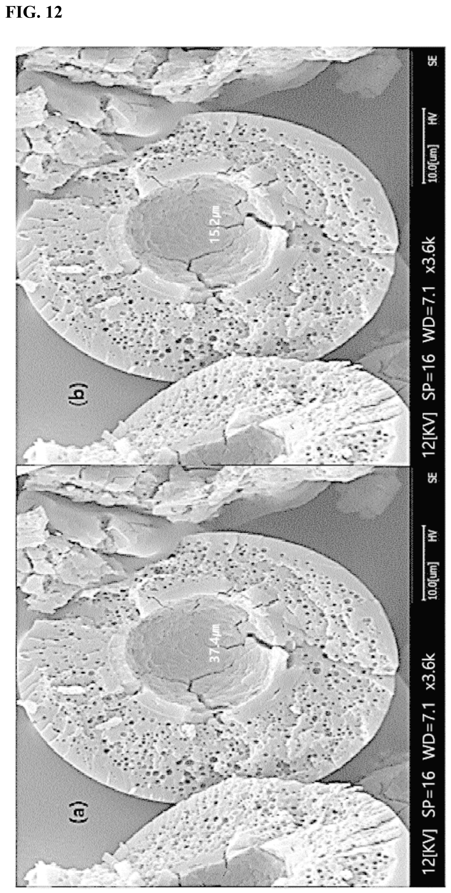

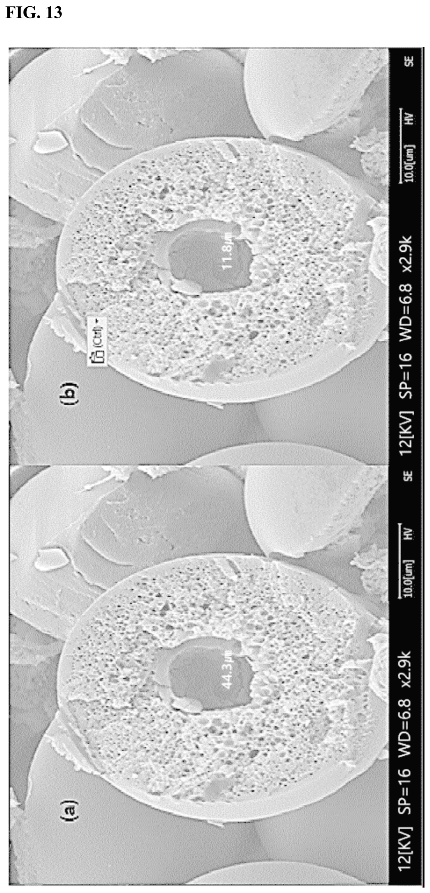

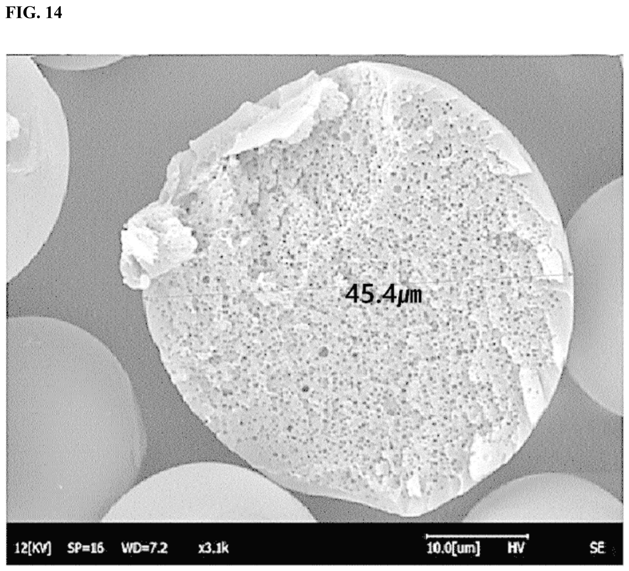

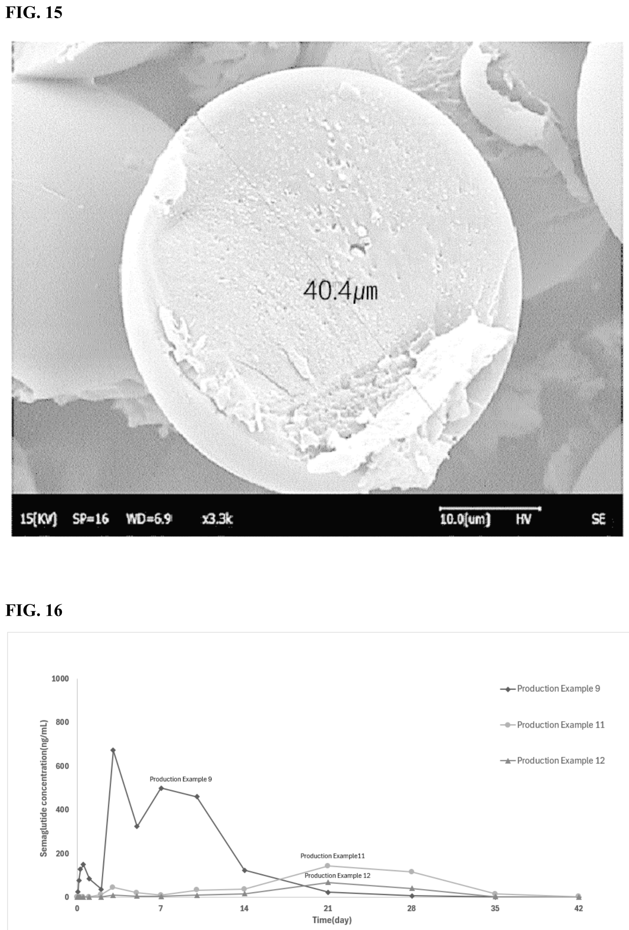

SEM images of the cross-sections for Production Examples 6 to 12 are as shown in FIGS. 9 to 15. FIGS. 14 and 15 show cross-sections of Production Examples 11 and 12, and it can be confirmed that no hole was formed inside the microparticles. When examining the cross-sections in FIGS. 9 to 13, it can be confirmed that a hole was formed in the center.

The results of measuring the diameters for Preparation Examples 6 to 12 are shown in Table 7 below:

[Table 7]

|  | Production Example 6 | Production Example 7 | Production Example 8 | Production Example 9 | Production Example 10 | Production Example 11 | Production Example 12 |
|---|---|---|---|---|---|---|---|
| Channeling agents (g) | 2.05 | 1.55 | 1.75 | 3.25 | 3.50 | 3.85 | 4.55 |
| DCM (g) | 2.50 | 2.00 | 2.40 | 3.30 | 4.10 | 4.80 | 6.75 |
| **Weight (%) of channeling agents \*** | **45.1** | **43.7** | **42.2** | **49.6** | **46.1** | **44.5** | **40.3** |
| Cross-sectional diameter (μm) | 37.7 | 40.2 | 50.1 | 37.4 | 44.3 | 45.4 | 40.4 |
| Inner diameter (μm) | 25.5 | 22.6 | 21.5 | 15.2 | 11.8 | 0 | 0 |

(continued)

|  | Production Example 6 | Production Example 7 | Production Example 8 | Production Example 9 | Production Example 10 | Production Example 11 | Production Example 12 |
|---|---|---|---|---|---|---|---|
| Value of [Equation 1] | 0.324 | 0.438 | 0.571 | 0.594 | 0.734 | 1.000 | 1.000 |

\* is the weight (%) of the channeling agents relative to the total weight of dichloromethane and the channeling agents. The value of Equation 1 is a value calculated by Equation 1 below:

$$[Equation \ 1]$$

$$(D_{sp} - D_p)/D_{sp}$$

wherein

$D_{sp}$ is the diameter ($\mu$m) of the cross-section of the microparticle, and

$D_p$ is the diameter ($\mu$m) of the hole formed in the center of the cross-section of the microparticle.

For Production Examples 9, 11 and 12, pharmacokinetic evaluation was conducted to determine the release of semaglutide. The test conditions are as follows:

- Male SD rats, 7 weeks old
- Ten SD rats per group (a total of 30 SD rats) were used, and the average value of the blood semaglutide concentrations was calculated for each measurement time.
- Subcutaneous administration (2.8 mg/kg)

The test results are shown in Table 8 below and FIG. 16:

[Table 8]

| Time (day) | Time (hour) | Production Example 9 | Production Example 11 | Production Example 12 |
|---|---|---|---|---|
| 0 | 0 | ND | ND | ND |
| 0.04 | 1 | 25.9303 | 0.2087 | BQL |
| 0.13 | 3 | 76.9545 | 0.9417 | 1.8977 |
| 0.25 | 6 | 128.2969 | 1.8053 | 2.5942 |
| 0.50 | 12 | 149.8705 | 1.6421 | 2.2119 |
| 1 | 24 | 84.4586 | 1.4529 | 1.1452 |
| 2 | 48 | 37.7296 | 13.1989 | 2.5393 |
| 3 | 72 | 673.1378 | 45.6030 | 9.8311 |
| 5 | 120 | 324.5922 | 21.2385 | 5.7687 |
| 7 | 168 | 500.6486 | 10.3236 | 4.7654 |
| 10 | 240 | 460.4982 | 33.2384 | 10.6622 |
| 14 | 336 | 124.3077 | 37.9153 | 17.5879 |
| 21 | 504 | 23.2738 | 143.5013 | 68.5863 |
| 28 | 672 | 7.5408 | 115.9498 | 41.3481 |
| 35 | 840 | 1.5128 | 15.0563 | 3.1216 |
| 42 | 1008 | BQL | 2.8632 | 1.0917 |
| AUC$_{last}$ (h·ng/mL) | | 127625.523 | 54644.176 | 21855.529 |

Referring to the test results above, it was confirmed that, in the case of Production Example 9, initial burst release of semaglutide did not occur, the release pattern of semaglutide increased significantly after day 2 and then gradually decreased, and semaglutide was released in a sustained manner for up to 6 weeks.

It was confirmed that, in the case of Production Example 11, the release pattern of a low concentration of semaglutide appeared initially after administration, the release pattern of semaglutide increased after day 14 and then gradually decreased, and semaglutide was released in a sustained manner for up to 6 weeks.

It was confirmed that Production Example 12 showed a semaglutide release pattern similar to that of Production Example 11 and showed a pattern in which semaglutide was released in a sustained manner for up to 6 weeks, but the concentration of semaglutide was lower, and the bioavailability was very low at about 17% of that of Production Example 9.

Based on the pK test results for Production Example 9 as described above, an accelerated release test was conducted on other Production Examples.

For the accelerated release test, microparticles with a dose equivalent to 5 mg semaglutide were used, and a release test solution was prepared by mixing 1X PBS buffer (pH 7.4), 0.06% (w/v) polysorbate 80, and 0.01% (w/v) sodium azide.

A shaking water bath was used as a dissolution tester, and a glass test vessel with a capacity of 120 ml was used as a dissolution test vessel. As operating conditions, shaking was performed horizontally in a direction perpendicular to the bottom of the vessel and performed 120 times (reciprocating) at 40°C.

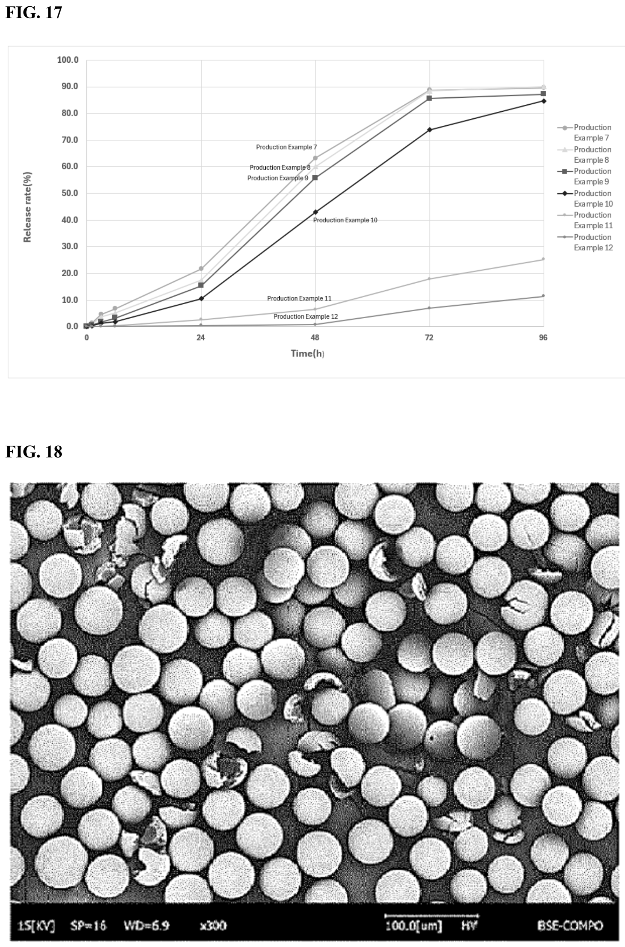

The test results are as shown in Table 9 below and FIG. 17:

[Table 9]

| Time (hour) | Production Example 7 | Production Example 8 | Production Example 9 | Production Example 10 | Production Example 11 | Production Example 12 |
|---|---|---|---|---|---|---|
| 0 | 0.0 | 0.0 | 0.0 | 0.0 | 0.0 | 0 |
| 1 | 1.3 | 0.8 | 0.6 | 0.4 | 0.1 | 0.1 |
| 3 | 4.6 | 3.6 | 1.8 | 1.3 | 0.3 | 0.1 |
| 6 | 6.8 | 5.2 | 3.3 | 1.9 | 0.4 | 0.2 |
| 24 | 21.7 | 17.5 | 15.4 | 10.6 | 2.7 | 0.5 |
| 48 | 63.2 | 60.1 | 55.8 | 43.0 | 6.5 | 0.8 |
| 72 | 88.8 | 88.4 | 85.6 | 73.9 | 17.9 | 6.9 |
| 96 | 89.6 | 90.1 | 87.2 | 84.7 | 25.2 | 11.4 |

Production Example 9 showed a release pattern that continuously increased from the initial stage after administration, and then showed a pattern in which the drug began to be released significantly after 24 hours and the release rate of semaglutide continuously increased.

Production Examples 7, 8, and 10 above showed release rates and release patterns similar to those of Production Example 9, and based thereon, it can be predicted that the microparticles have similar bioavailability and drug release patterns in *in vivo* tests.

On the other hand, in the case of Production Examples 11 and 12, the point in time at which the drug was significantly released was relatively late even in the accelerated release test, which corresponds to the results of the above-described pK test.

In the case of Production Example 6, despite the fact that semaglutide used in the process of preparing the oil phase solution exceeded 30 wt%, the encapsulation efficiency of semaglutide in the microparticles was low at 71.4%. In addition, because the amount of channeling agents used was large, the value of Equation 1 was 0.324, and thus the diameter of the hole in the microparticle was excessively large compared to the diameter of the cross-section, so that the microparticles easily broke down during the filling process as shown in FIG. 8. The broken microparticles were excluded from the pK test because they could significantly affect the release pattern of semaglutide when administered *in vivo*.

The results of analyzing the particle sizes of the microparticles of Production Examples 6 to 12 are shown in Table 10 below:

[Table 10]

| | Production Example 6 | Production Example 7 | Production Example 8 | Production Example 9 | Production Example 10 | Production Example 11 | Production Example 12 |
|---|---|---|---|---|---|---|---|
| D10 | 31.37 | 30.20 | 39.59 | 32.52 | 38.74 | 30.58 | 32.17 |
| D20 | 32.75 | 31.92 | 41.87 | 33.71 | 40.23 | 31.84 | 34.40 |
| D30 | 33.91 | 33.21 | 43.65 | 34.79 | 41.55 | 32.77 | 36.19 |

(continued)

|  | Production Example 6 | Production Example 7 | Production Example 8 | Production Example 9 | Production Example 10 | Production Example 11 | Production Example 12 |
|---|---|---|---|---|---|---|---|
| D40 | 35.01 | 34.42 | 45.07 | 35.85 | 42.86 | 33.58 | 37.76 |
| D50 | 36.16 | 35.63 | 46.38 | 36.93 | 44.16 | 34.40 | 39.20 |
| D60 | 37.39 | 36.94 | 47.64 | 38.09 | 45.51 | 35.23 | 40.66 |
| D70 | 38.79 | 38.56 | 49.00 | 39.33 | 46.93 | 36.21 | 42.22 |
| D80 | 40.44 | 40.57 | 50.50 | 40.70 | 48.48 | 37.42 | 44.18 |
| D90 | 42.62 | 43.41 | 52.37 | 42.39 | 50.35 | 39.62 | 47.49 |
| D95 | 44.11 | 45.92 | 54.54 | 43.55 | 51.62 | 41.58 | 50.38 |
| Span value | 0.31 | 0.37 | 0.28 | 0.27 | 0.26 | 0.26 | 0.39 |

Referring to the above particle size analysis results, it can be confirmed that the microparticles of Production Examples 6 to 12 are very uniform particles with D50 values of 36.16 $\mu$m, 35.63 $\mu$m, 46.38 $\mu$m, 36.93 $\mu$m, 44.16 $\mu$m, 34.40 $\mu$m and 39.20 $\mu$m, respectively, and span values of 0.31, 0.37, 0.28, 0.27, 0.26, 0.26 and 0.39, respectively.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

**Industrial Applicability**

The present invention relates to a microparticle containing semaglutide and a method for producing the same.

**Claims**

1. A microparticle containing semaglutide or a pharmaceutically acceptable salt thereof,

   wherein the microparticle comprises semaglutide or the pharmaceutically acceptable salt thereof and a biodegradable polymer, and
   a cross-section of the microparticle has a shape with a hole formed in a center thereof, and has a value of 0.35 to 0.80 as calculated by Equation 1 below:

$$[\text{Equation 1}]$$

$$(D_{sp} - D_p)/D_{sp}$$

   wherein

      $D_{sp}$ is a diameter ($\mu$m) of the cross-section of the microparticle, and
      $D_p$ is a diameter ($\mu$m) of the hole formed in the center of the cross-section of the microparticle.

2. The microparticle of claim 1, wherein the microparticle comprises semaglutide or the pharmaceutically acceptable salt thereof and the biodegradable polymer at a weight ratio of 1:8 to 1:20.

3. The microparticle of claim 1, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

4. The microparticle of claim 3, wherein the polylactide-co-glycolide has a monomer ratio of lactide: glycolide of 50:50 to 85:15.

5. The microparticle of claim 1, wherein the biodegradable polymer has an intrinsic viscosity of 0.1 dl/g to 0.5 dl/g.

6. The microparticle of claim 1, wherein the microparticle releases semaglutide or the pharmaceutically acceptable salt thereof in a sustained manner for 4 weeks or more after being injected *in vivo.*

7. The microparticle of claim 1, wherein the microparticle has a span value of 1.2 or less.

8. The microparticle of claim 1, wherein the microparticle has an average diameter of 30 to 70 $\mu$m.

9. A method for producing microparticles containing semaglutide or a pharmaceutically acceptable salt thereof, comprising:

a step of preparing an oil phase solution containing semaglutide or the pharmaceutically acceptable salt thereof and a biodegradable polymer;
a step of preparing a water phase solution by dissolving a surfactant in water;
a step of forming an emulsion by mixing the oil phase solution and the water phase solution;
a step of collecting the emulsion into the water phase solution to remove residual solvent; and
a step of washing and freeze-drying the emulsion from which the residual solvent has been removed, thereby producing the microparticles containing semaglutide or the pharmaceutically acceptable salt thereof,
wherein a cross-section of the microparticle has a shape with a hole formed in a center thereof, and has a value of 0.35 to 0.80 as calculated by Equation 1 below:

[Equation 1]

$$(D_{sp} - D_p)/D_{sp}$$

wherein

$D_{sp}$ is a diameter ($\mu$m) of the cross-section of the microparticle, and
$D_p$ is a diameter ($\mu$m) of the hole formed in the center of the cross-section of the microparticle.

10. The method of claim 9, wherein the oil phase solution is prepared by mixing a first oil phase solution containing semaglutide and a second oil phase solution containing the biodegradable polymer, wherein the first oil phase solution contains semaglutide or the pharmaceutically acceptable salt thereof and a channeling agent.

11. The method of claim 10, wherein the channeling agent is selected from the group consisting of methanol, ethanol, propanol, butanol, hydrochloric acid (HCl), nitric acid (HNO$_3$), sulfuric acid (H$_2$SO$_4$), acetic acid (CH$_3$COOH), boric acid (H$_3$BO$_3$), carbonic acid (H$_2$CO$_3$), dimethyl sulfoxide (C$_2$H$_6$OS), acetonitrile (C$_2$H$_3$N), ethyl acetate (C$_4$H$_8$O$_2$), and mixtures thereof.

12. The method of claim 10, wherein the channeling agent is contained in an amount of 30 wt% to 60 wt% based on the total weight of an organic solvent of the second oil phase solution.

**FIG. 1**

FIG. 2

**FIG. 3**

**FIG. 4**

**FIG. 5**

**FIG. 6**

**FIG. 7**

FIG. 8

**FIG. 9**

**FIG. 10**

**FIG. 11**

**FIG. 12**

**FIG. 13**

**FIG. 14**

**FIG. 15**

**FIG. 16**

**FIG. 17**

**FIG. 18**

## INTERNATIONAL SEARCH REPORT

| International application No. |
| --- |
| **PCT/KR2024/010766** |

| A. | CLASSIFICATION OF SUBJECT MATTER |
| --- | --- |

**A61K 9/16**(2006.01)i; **A61K 9/00**(2006.01)i; **A61K 38/26**(2006.01)i

According to International Patent Classification (IPC) or to both national classification and IPC

| B. | FIELDS SEARCHED |
| --- | --- |

Minimum documentation searched (classification system followed by classification symbols)

A61K 9/16(2006.01); A61K 31/00(2006.01); A61K 38/26(2006.01); A61L 27/18(2006.01); B01J 13/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세마글루타이드(semaglutide), 생분해성 고분자(biodegradable polymer), 마이크로 입자(microparticle), 중공(hollow)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2021-0103985 A (G2GBIO, INC.) 24 August 2021 (2021-08-24)<br>See paragraphs [0039], [0050], [0052], [0067], [0101]-[0102] and [0141]; claims 1-2 and 9; and figure 3. | 1-12 |
| Y | KHAREL, Sharad et al. Hollow Microparticles as a Superior Delivery System over Solid Microparticles for the Encapsulation of Peptides. Pharm Res. 02 August 2018 (Online), vol. 35, 185, pp. 1-10.<br>See abstract; page 2, right column and page 4, left column; and figures 1 and 5. | 1-12 |
| Y | KR 10-2018-0009005 A (KOREA RESEARCH INSTITUTE OF BIOSCIENCE AND BIOTECHNOLOGY et al.) 25 January 2018 (2018-01-25)<br>See claims 1 and 6. | 11 |
| A | WO 2016-089309 A1 (NANYANG TECHNOLOGICAL UNIVERSITY) 09 June 2016 (2016-06-09)<br>See entire document. | 1-12 |
| A | KR 10-2047983 B1 (G2GBIO, INC.) 22 November 2019 (2019-11-22)<br>See entire document. | 1-12 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 November 2024** | **04 November 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/010766**

| Patent document cited in search report | | | Publication date (day/month/year) | Patent family member(s) | | | Publication date (day/month/year) |
|---|---|---|---|---|---|---|---|
| KR | 10-2021-0103985 | A | 24 August 2021 | CN | 115484935 | A | 16 December 2022 |
| | | | | EP | 4104820 | A2 | 21 December 2022 |
| | | | | EP | 4104820 | A4 | 17 April 2024 |
| | | | | JP | 2023-513751 | A | 03 April 2023 |
| | | | | JP | 7548600 | B2 | 10 September 2024 |
| | | | | KR | 10-2022-0035361 | A | 22 March 2022 |
| | | | | KR | 10-2024-0122403 | A | 12 August 2024 |
| | | | | KR | 10-2375262 | B1 | 16 March 2022 |
| | | | | US | 2023-0096928 | A1 | 30 March 2023 |
| | | | | WO | 2021-162532 | A2 | 19 August 2021 |
| | | | | WO | 2021-162532 | A3 | 30 September 2021 |
| KR | 10-2018-0009005 | A | 25 January 2018 | KR | 10-1826910 | B1 | 08 February 2018 |
| WO | 2016-089309 | A1 | 09 June 2016 | | None | | |
| KR | 10-2047983 | B1 | 22 November 2019 | CN | 110709067 | A | 17 January 2020 |
| | | | | CN | 110709067 | B | 14 October 2022 |
| | | | | EP | 3586828 | A1 | 01 January 2020 |
| | | | | EP | 3586828 | A4 | 13 January 2021 |
| | | | | EP | 3586828 | B1 | 02 March 2022 |
| | | | | JP | 2020-521805 | A | 27 July 2020 |
| | | | | JP | 2022-023219 | A | 07 February 2022 |
| | | | | JP | 7468909 | B2 | 16 April 2024 |
| | | | | KR | 10-2019-0064509 | A | 10 June 2019 |
| | | | | US | 11311854 | B2 | 26 April 2022 |
| | | | | US | 2020-0298196 | A1 | 24 September 2020 |
| | | | | WO | 2019-108030 | A1 | 06 June 2019 |

Form PCT/ISA/210 (patent family annex) (July 2022)

**REFERENCES CITED IN THE DESCRIPTION**

**Patent documents cited in the description**

- WO 9943708 A
- WO 0034331 A
- WO 0069911 A
- KR 1020220170782 A1